(19) Europäisches Patentamt
European Patent Office
Office européen des brevets

(11) Veröffentlichungsnummer: **0 034 368**
**B1**

# (12) EUROPÄISCHE PATENTSCHRIFT

(45) Veröffentlichungstag der Patentschrift: **20.06.84**

(51) Int. Cl.³: **C 07 J 41/00**

(21) Anmeldenummer: **81101119.6**

(22) Anmeldetag: **17.02.81**

## (54) Verfahren zur Umwandlung von Steroid-20-Carbonsäureamiden in die entsprechenden Steroid-20-Carbamate.

(30) Priorität: **19.02.80 US 122395**

(43) Veröffentlichungstag der Anmeldung: **26.08.81 Patentblatt 81/34**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen:
**ORGANIC REACTIONS, Band III, 1947, Kapitel 7, Seiten 267-286 Aug. John Wiley & Sons, Inc. New York, U.S.A. E.S. WALLIS et al.: "The Hofmann reaction"**

(73) Patentinhaber: **HENKEL CORPORATION,** ***7900 West 78th Street*****, Minneapolis Minnesota 55435 (US)**

(72) Erfinder: **Krbechek, Leroy O., 2041 Orkla Drive, Minneapolis Minnesota 55427 (US)**

(74) Vertreter: **Fues, Johann Friedrich, Dr. et al, Deichmannhaus am Hauptbahnhof, D-5000 Köln 1 (DE)**

**EP 0 034 368 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung beschreibt eine wichtige Stufe aus der chemischen Transformation grosstechnisch zugänglicher BNC-Verbindungen zu Progesteron und verwandten Verbindungen vom Progesteron-Typ.

Die offengelegte Europäische Patentanmeldung 79101036.6 (4913) beschreibt u.a. ein Verfahren zur Herstellung von 3-Oxo-pregna-4-en-20-carbonsäure($\Delta^{4}$-BNC) und/oder 3-Oxo-pregna-1,4-dien-20-carbonsäure($\Delta^{1,4}$-BNC) durch mikrobiellen Seitenkettenabbau an 17-C-Seitenketten-Steroidsubstraten natüurlichen Ursprungs. Die genannten BNC-Verbindungen sind auf diesem Wege im grosstechnischen Massstab wohlfeil erhältlich. In der Europäischen Patentanmeldung 81100146.0 (33439) wird eine Modifikation dieses Verfahrens geschildert, wodurch eine weitere homologe BNC-Verbindung, nämlich die 3-Oxo-pregna-1,4,17(20)-trien-20-carbonsäure($\Delta^{1,4,17(20)}$-BNC), zugänglich wird. Die US-PS 3 994 933 beschreibt die Herstellung von 3-Oxo-pregna-4,17(20)-dien-20-carbonsäure($\Delta^{4,17(20)}$-BNC) durch mikrobiellen Seitenkettenabbau an Substraten natürlichen Ursprungs.

Die hier genannten Steroid-20-Carbonsäureverbindungen sind in den folgenden Formelbildern A bis D dargestellt, worin der Rest X jeweils die Carboxylgruppe COOH bedeutet und A $\Delta^{1,4}$-BNC, B $\Delta^{4}$-BNC, C $\Delta^{1,4,17}$-BNC und D $\Delta^{4,17}$-BNC entsprechen.

A

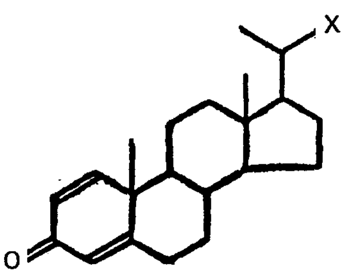

B

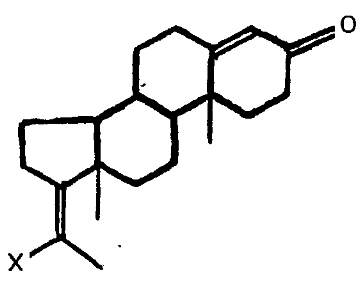

C

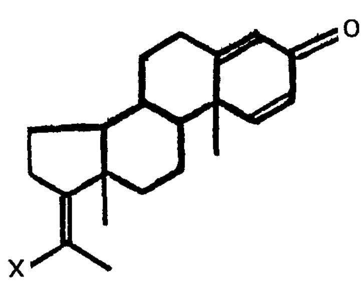

D

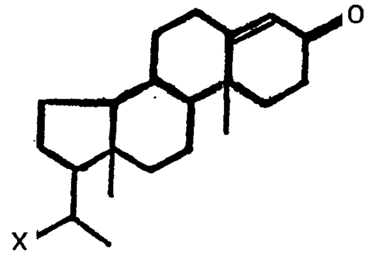

Zur Umwandlung dieser BNC-Ausgangsverbindungen zu Kommponenten des Progesterontyps bedarf es des Abbaus der Carboxylgruppe in C-20 und der Einführung einer Oxogruppe in die 20-Stellung des Steroids. Die Erfindung beschreibt einen wichtigen Teilaspekt einer solchen mehrstufigen Umwandlung, wobei sich das erfindungsgemässe Verfahren durch besondere Einfachheit und hohe Ausbeuten auszeichnet. Das erfindungsgemässe Verfahren macht insbesondere chemische Umwandlungen am Seitenkettensubstituenten in C-17 möglich, ohne dass die im Steroidsystem vorliegenden Doppelbindungen angegriffen würden und/oder ohne dass ein Schutz der 3-Ketofunktion im A-Ring des Steroidsystems erforderlich würde.

Die Erfindung hat sich die Aufgabe gestellt, ein Verfahren zu entwickeln, durch das BNC-Säureamide der formelmässig in A bis D dargestellten Art (X = $CONH_2$) ohne besondere Schutzmassnahmen zur restlichen Steroid-Ringstruktur mit hohen Ausbeuten in die entsprechenden Carbamate umgewandelt werden können (X = NHCOOR). Diese Carbamate können dann ihrerseits Ausgangsmaterial für die weitere Umwandlung der Position 20 zur gewünschten 20-Oxogruppe sein.

Gegenstand der Erfindung ist dementsprechend ein Verfahren zur Herstellung von Steroid-20-Carbamaten aus der Gruppe 3-Oxo-pregn-4-en-20-carbamat, 3-Oxo-pregna-1,4-dien-20-carbamat, 3-Oxo-pregna-4,17(20)-dien-20-carbamat und/oder 3-Oxo-pregna-1,4,17(20)-trien-20-carbamat aus den entsprechenden Steroid-20-Carboxamiden, wobei das Verfahren dadurch gekennzeichnet ist, dass man

a) die entsprechenden Steroid-20-Säureamide mit Brom bzw. einer Brom liefernden Verbindung zum entsprechenden Bromamid umsetzt,

b) das gebildete Bromamid unter Bildung des 20-Isocyanats mit einer starken Base behandelt,

c) und das erhaltene Reaktionsprodukt mit einem einwertigen Alkohol zum Steroid-20-Carbamat umsetzt.

Gegenstand der parallelen Europäischen Patentanmeldung 81101117.0 (34366) («Verfahren zur Herstellung von Steroid-20-Carbamatverbindungen», US-Priorität vom 19. Februar 1980) ist ein entsprechendes Verfahren, bei dem unter Verwendung von N-Bromsuccinimid oder N-Bromphthalimid aus den Steroid-20-BNC-Carboxamiden mittels einer Transhalogenierung zunächst die Bildung von Steroid-20-N-Bromamiden erfolgt, die dann in analoger Weise durch Behandlung mit einer starken Base und Umsetzung mit Alkoholen in die Carbamate umgewandelt werden. Das Verfahren dieser parallelen Europäischen Patentanmeldung wird im Rahmen der vorliegenden Lehre nicht noch einmal beansprucht.

Die erfindungsgemäss als Ausgangsmaterialien einzusetzenden Steroid-20-Carbonsäureamide können aus den entsprechenden BNC-Verbindungen mittels Halogenierung zum Säurehalogenid — insbesondere Säurechlorid — und dessen anschliessende Umsetzung mit Ammoniak zum Säureamid gewonnen werden. Einzelheiten zu diesen Verfahrensstufen, die eine Halogenierung mir vorzugsweise Thionylchlorid und die anschliessende Umsetzung des gebildeten Säurechlorids mit Ammoniak oder Ammoniak liefernden Verbindungen umfassen, sind der Europäischen Patentanmeldung 81100145.2 (34248) und der parallelen Europäischen Patentanmeldung 81101114.7 (34364) («Neue 20-substituierte 3-Oxo-pregna-4-en-steroidverbindungen und Verfahren zu ihrer Herstellung», US-Priorität vom 19. Februar 1980) der gleichen Anmelderin zu entnehmen.

Die Steroid-20-Carboxamidverbindung wird erfindungsgemäss in einer ersten Stufe mit Brom bzw. Brom liefernden Verbindungen zum entsprechenden N-Bromamid umgesetzt. Eine geeignete Quelle für Brom ist elementares Brom, wenngleich auch andere Materialien, die in der Lage sind, Brom oder ein positives Bromion zu bilden, statt dessen eingesetzt werden können. Die Umsetzung des Säureamids mit Brom erfolgt zweckmässigerweise in Lösungsmitteln. Geeignet sind beispielsweise aprotische Lösungsmittel, wie halogenierte Kohlenwasserstoffreste, beispielsweise Methylenchlorid oder Ethylendichlorid. In einer besonderen Ausführungsform der Erfindung können Alkohole, insbesondere die zur Carbamatbildung benötigten Alkohole, als reaktives Lösungsmittel eingesetzt werden. Möglich ist insbesondere weiterhin die Verwendung von gemischten Lösungsmittelsystemen, die sowohl den zur Carbamatbildung erforderlichen Alkohol als daneben aprotische Lösungsmittel der zuvor genannten Art enthalten. Das Lösungsmittelgemisch kann den Alkohol zum Co-Lösungsmittel, beispielsweise in Mengen von 15 : 1 bis 1 : 10 enthalten, wobei jedoch auf jeden Fall Alkohol in hinreichender Menge zur Bildung des Carbamats eingesetzt werden sollte.

Zur Bildung des entstehenden Bromwasserstoffs wird dem Reaktionsgemisch eine basische Komponente zugesetzt. Zweckmässig kann es sein, auch für diesen Neutralisationsschritt die gleiche starke Base zu verwenden, wie sie im nachfolgenden Reaktionsschritt zur Abspaltung des Bromwasserstoffs Verwendung findet. In dieser Ausführungsform der Erfindung kommt damit die starke Base in wenigstens 2fach äquivalenter Menge — bezogen auf eingesetzte Steroid-Carboxamidverbindung — zum Einsatz. Es kann dabei bevorzugt sein, dem Reaktionsansatz von Anfang an die gesamte Menge der starken Base zuzusetzen.

Als starke Basen geeignet sind beispielsweise Natriumhydroxid, Kaliumhydroxid oder die entsprechenden Alkoholate, insbesondere Natriummethoxid, bzw. Mischungen dieser Komponenten.

In einer weiteren Ausführungsform der Erfindung kann es zweckmässig sein, zur Unterstützung der Reaktion einen Urethan bildenden Katalysator zuzusetzen, der dem Reaktionsgemisch auch schon von Anfang an zugegeben werden kann. Ein geeigneter Urethan bildender Katalysator ist Dibutylzinndilaurat.

Die Reaktion wird vorzugsweise unter Inertgas in Abwesenheit von Sauerstoff durchgeführt. Stickstoff ist ein geeignetes Inertgas. Zweckmässigerweise wird dabei schon der Reaktionsansatz, der wenigstens die Carboxamidverbindung im Lösungsmittel enthält, von Sauerstoff befreit, beispielsweise indem man den Reaktionsansatz von einem Stickstoffstrom durchspülen lässt.

Dem Reaktionsgemisch wird dann Brom bzw. die Brom liefernde Verbindung — bevorzugt elementares Brom — zugesetzt. Die eingesetzte Brommenge entspricht wenigstens dem Äquivalenzbetrag, vorzugsweise wird mit leichtem Bromüberschuss gearbeitet. Die eingesetzte Brommenge kann somit wenigstens 1,1 Äquivalente, insbesondere wenigstens etwa 1,3 Äquivalente, bezogen auf den theoretisch erforderlichen Betrag, ausmachen.

Brom bzw. die Brom lefernde Verbindung wird der Reaktionsmischung im allgemeinen während eines Zeitraums von einigen Minuten zugesetzt. Hierbei ist sicherzustellen, dass die Reaktion nicht zu stürmisch verläuft. In diesem Zusammenhang ist zu vermerken, dass Überraschendes für das erfindungsgemässe Verfahren schon darin liegt, dass das Brom sich nicht auch an Doppelbindungen anlagert, die in den Steroid-Ausgangsmaterialien vorliegen, sei es im Ringsystem und/oder in der 17(20)-Stellung des Steroidmoleküls. Zu berücksichtigen ist in diesem Fall weiterhin, dass zur Bildung der Bromamidgruppierung die Verwendung der genannten starken Basen erforderlich ist, da sich nur in ihrer Gegenwart die gewünschte Bromamidverbindung bildet. Es ist überraschend, dass auch aus dieser Seite der aus der Natur der Sache heraus bedingten Notwendigkeiten für den Reaktionsablauf keine unerwünschten Nebenreaktionen an der komplexen Steroidstruktur in nennenswertem Ausmass eintreten bzw. ausgelöst werden. Es war für den Fachmann nicht vorherzusehen, dass der erfindungsgemäss vorgesehene Reaktionsablauf an Steroidverbindungen der erfindungsgemäss eingesetzten Art weitgehend selektiv eintritt, ohne dass Nebenreaktionen stören.

Nach Zusatz des Broms wird das Reaktionsgemisch für einen hinreichenden Zeitraum, beispielsweise für etwa 1 Stunde, gerührt, um die Reaktion zu vervollständigen. Anschliessend wird die Reaktionsmischung mit einer schwachen Säure, beispielsweise Essigsäure, angesäuert und dann in üblicher Weise aufgearbeitet. So wird die Lösungsmittelphase beispielsweise bei verringertem Druck abgezogen. Wird Alkohol als reaktives Lösungsmittel allein oder in Abmischung mit anderen Lösungsmitteln eingesetzt, so fällt unmittelbar als Rohprodukt das entsprechende Steroid-20-Carbamat an. In der erfindungsgemäss besonders bevorzugten Ausführungsform wird das Gesamtverfahren in einem Zuge in praktisch einem Verfahrensschritt durchgeführt.

Die Verfahrenstemperatur liegt üblicherweise im Bereich von etwa 0°C bis 60°C. Es kann zweckmässig sein, Temperaturen im Bereich der Raumtemperatur nicht wesentlich zu überschreiten.

Die Aufarbeitung des rohen Carbamatprodukts erfolgt beispielsweise durch Waschen mit einem organischen Lösungsmittel, wie Methylenchlorid, und anschliessend mit Wasser. Zweckmässigerweise kann auch eine Waschstufe mit einem Thiosulfatsalz eingeschlossen sein. Das gewaschene Verfahrensprodukt wird getrocknet und eventuell noch vorliegendes Lösungsmittel z.B. durch Verdampfen unter verringertem Druck entfernt.

Bevorzugte Alkohole zur Carbamatbildung enthalten nicht mehr als 10 C-Atome. Niedere Alkanole mit insbesondere bis zu 5 C-Atomen sind besonders bevorzugt. Geeignete Alkohole sind insbesondere Methanol und tert.-Butanol, aber auch Ethanol, die Propanole sowie die anderen $C_4$-Alkohole.

Aus dem Stand der Technik zur chemischen Transformation von Steroid-Verbindungen zu Progesteron wird verwiesen auf Canadian Journal of Chemistry, Band 34 (1956), S. 982-990, sowie Helvetica Chimica Acta, Band XXXII, Teil VI (1949), Nr. 255, S. 1922-1933, sowie a.a.O., Band XXXII, Teil V (1949), Nr. 233, S. 1764-1769. Verwiesen wird weiterhin auf JACS, Band LXX (1948), Nr. 3, S. 887-892, sowie Helvetica Chimica Acta, Band XXXII, Teil V (1949), Nr. 232, S. 1758-1763. Verwiesen sei schliesslich noch auf die US-PS 3 519 658.

*Beispiel*

343 g 3-Oxo-pregna-4-en-20-carbonsäureamid werden in 7,5 l Methanol gelöst, das 162 g Natriummethoxid und 750 ml Methylenchlorid enthält. 10 g Dibutylzinndilaurat weerden als Urethanbildungskatalysator zugesetzt. Das Reaktionsgemisch wird dann unter eine Stickstoffatmosphäre gebracht und bei Raumtemperatur für etwa 15 Minuten gerührt, woraufhin 240 g elementares Brom während eines Zeitraums von etwa 45 Minuten zugesetzt werden.

Die angefallene Reaktionsmischung wird für etwa eine weitere Stunde gerührt und anschliessend mit 250 ml Essigsäure angesäuert. Anschliessend wird die Lösungsmittelphase unter vermindertem Druck entfernt. Der Rückstand wird in Methylenchlorid aufgenommen und nacheinander mit Wasser, Natriumthiosulfatlösung und wieder Wasser gewaschen. Anschliessend wird mit Calciumsulfat getrocknet, dann wird das Lösungsmittel bei verringertem Druck entfernt. Erhaltenn werden 390 g eines Rohprodukts, das zu mehr als 70% der Theorie aus dem 20-Methylcarbamat besteht, das dem eingesetzten Steroid-20-Säureamid entspricht. Im Reaktionsrohprodukt wird nur noch ein geringer Anteil an nicht umgesetztem Amid gefunden. Die mehrfache Wiederholung des Ansatzes mit Natriumhydroxid und Kaliumhydroxid als starke Base führt ebenfalls zum 20-Methylcarbamat. Natriummethoxid erweist sich jedoch als die beste basische Reaktionskomponente.

Vergleichbare Ausbeuten an entsprechendem Reaktionsprodukt werden erhalten, wenn als Steroidausgangsmaterial 3-Oxo-pregna-1,4-dien-20-carbonamid, 3-Oxo-pregna-1,4,17(20)-trien-20-carboxamid oder 3-Oxo-pregna-4,17(20)-dien-20-carboxamid eingesetzt werden. Dabei ist in den beiden zuletzt genannten Fällen auf einen sorgfältigen Ausschluss von Wasser aus dem Reaktionsgemisch zu achten.

**Patentansprüche**

1. Verfahren zur Herstellung von Steroid-20-Carbamatverbindungen aus der Gruppe 3-Oxo-pregn-4-en-20-carbamat, 3-Oxo-pregna-1,4-dien-20-carbamat, 3-Oxo-pregna-4,17(20)-dien-carbamat und/oder 3-Oxo-pregna-1,4,17(20)-trien-20-carbamat aus den entsprechenden Steroid-20-Carbonsäureamiden, dadurch gekennzeichnet, dass man

a) das Steroid-20-Carbonsäureamid mit Brom bzw. einer Brom liefernden Verbindung zum entsprechenden Bromamid umsetzt,

b) das gebildete Bromamid unter Bildung des Steroid-20-Isocyanats mit einer starken Base behandelt und

c) dieses mit einem einwertigen Alkohol umsetzt.

2. Verfahren nach Anspruch 1, dadurch gekennzeichnet, dass man das Mehrstufenverfahren in einem Zuge ohne Isolierung der Reaktionszwischenprodukte durchführt.

3. Verfahren nach Ansprüchen 1 und 2, dadurch gekennzeichnet, dass man mit Alkoholen mit nicht mehr als 10 C-Atomen, insbesondere mit niederen Alkanolen mit vorzugsweise bis zu 5 C-Atomen, als Alkoholkomponente arbeitet.

4. Verfahren nach Ansprüchen 1 bis 3, dadurch gekennzeichnet, dass man als starke Base Natriumhydroxid, Kaliumhydroxid und/oder die entsprechenden Alkoxide, insbesondere Natriummethoxid, einsetzt.

5. Verfahren nach Ansprüchen 1 bis 4, dadurch gekennzeichnet, dass man den Alkohol — ggf. in Abmischung mit inerten Lösungsmitteln, wie Halogenkohlenwasserstoffen — als reaktives Lösungsmittel einsetzt.

6. Verfahren nach Ansprüchen 1 bis 5, dadurch gekennzeichnet, dass man bei Temperaturen von etwa 0°C bis 60°C, insbesondere bei Temperaturen nicht wesentlich über Raumtemperatur, und in einer inerten Atmosphäre arbeitet.

7. Verfahren nach Ansprüchen 1 bis 6, dadurch gekennzeichnet, dass man dem Reaktionsgemisch einen Urethanbildungskatalysator, wie Dibutylzinndilaurat, zusetzt.

**Claims**

1. A process for preparing a steroid-20-carbamate compound selected from the group consisting of 3-oxo-pregn-4-ene-20-carbamate, 3-oxo-pregna-1,4-diene-20-carbamate, 3-oxo-pregna-4,17(20)-diene-20-carbamate, and/or 3-oxo-pregna-1,4,17-(20)-triene-20-carbamate from the respective steroid-20-carboxylic acid amide, characterized in that

a) the steroid-20-carboxylic acid amide is reacted with bromine or a bromine-supplying compound, respectively, to form the corresponding bromoamide;

b) the obtained bromoamide is treated with a strong base to form the steroid-20-isocyanate; and

c) the latter product is reacted with a monohydric alcohol.

2. The process according to claim 1, characterized in that the multi-stage process is carried out non-

stop without isolating the intermediate reaction products.

3. The process according to claims 1 and 2, characterized in that alcohols having not more than 10 carbon atoms, more particularly lower alkanols having preferably up to 5 carbon atoms, are used as the alcohol component.

4. The process according to claims 1 to 3, characterized in that sodium hydroxide, potassium hydroxide, and/or the corresponding alkoxides, more specifically sodium methoxide, are used as the strong base.

5. The process according to claims 1 to 4, characterized in that the alcohol — optionally in admixture with inert solvents, such as halocarbons — is used as a reactive solvent.

6. The process according to claims 1 to 5, characterized in that there is operated at a temperature of from 0°C to 60°C, and preferably at a temperature which is not substantially higher than the room temperature, and in an inert atmosphere.

7. The process according to claims 1 to 6, characterized in that an urethane-formation catalyst, such as dibutyltin dilaurate, is added to the reaction mixture.

**Revendications**

1. Procédé de préparation de carbamates-20 de stéroïdes choisis dans l'ensemble formé par du carbamate-20 d'oxo-3 pregnène-4, du carbamate-20 d'oxo-3 pregnadiène-1,4, du carbamate-20 d'oxo-3 pregnadiène-4,17(20) et/ou du carbamate-20 d'oxo-3 pregnatriène-1,4,17(20) à partir des carboxamides-20 de stéroïdes correspondants, procédé caractérisé en ce que:

a) on fait réagir le carboxamide-20 de stéroïde avec du brome ou avec un composé fournissant du brome, pour obtenir le bromamide correspondant,

b) on traite par une base forte le formamide ainsi formé, en formant l'isocyanate-20 de stéroïde, et

c) on fait réagir ce dernier avec un monoalcool.

2. Procédé selon la revendication 1, caractérisé en ce qu'on effectue le procédé à plusieurs étapes en opérant d'un seul trait sans isoler les produits intérmédiaires de réaction.

3. Procédé selon les revendications 1 et 2, caractérisé en ce qu'on travaille avec des alcools n'ayant pas plus de 10 atomes de carbone, notamment avec des alcools inférieurs ayant de préférence jusqu'à 5 atomes de carbone, comme constituants alcools.

4. Procédé selon les revendications 1 à 3, caractérisé en ce qu'on utilise comme base forte l'hydroxyde de sodium, l'hydroxyde de potassium et/ou les alcoolates correspondants, notamment le méthylate de sodium.

5. Procédé selon les revendications 1 à 4, caractérisé en ce qu'on utilise l'alcool, éventuellement en mélange avec des solvants intertes, comme des hydrocarbures halogénés, comme solvant réactif.

6. Procédé selon les revendications 1 à 5, caractérisé en ce qu'on travaille à des températures d'environ 0°C à 60°C, notamment à des températures n'excédant pas notablement la température ambiante, et en atmosphère inerte.

7. Procédé selon les revendications 1 à 6, caractérisé en ce qu'on ajoute au mélange réactionnel un catalyseur de formation d'uréthane, comme du dilaurate de dibutylétain.